# EUROPEAN PATENT APPLICATION

(11) **EP 4 822 027 A1**
(43) Date of publication of application: **14.10.2026**
(21) Application number: 24901143.8
(22) Date of filing: 06.12.2024
(51) Int. Cl.: C12N 15/86

(54) **ADENO-ASSOCIATED VIRUS VARIANTS CAPABLE OF BRAIN ASTROCYTE-SPECIFIC GENE TRANSFER AND APPLICATIONS THEREOF**

(30) Priority: 06.12.2023 KR 20230175840
(71) Applicant: GLUGENE THERAPEUTIC INC., Daejeon 34109 (KR)
(72) Inventor: JANG, Jae-Hyung, Seoul 03722 (KR); SHIN, Jeong Eun, Seoul 04124 (KR); OH, Seokmin, Bucheon-si, Gyeonggi-do 14540 (KR); KIM, Miri, Seoul 04315 (KR); LEE, Moon Sue, Seoul 03722 (KR); KANG, Changmin, Seoul 03722 (KR); CHOI, Hongyoung, Seoul 03722 (KR)
(74) Representative: Graf von Stosch Patentanwaltsgesellschaft mbH
(86) International application number: PCT/KR2024/019997
(87) International publication number: WO 2025/121957

(57) **Abstract**

The present disclosure relates to adeno-associated virus variants capable of brain astrocyte-specific gene transfer. The administration of a recombinant viral vector comprising nucleic acid encoding a mutant of an AAV capsid protein according to the present disclosure allows a therapeutic gene to be transferred to and expressed in specific brain astrocytes, and thus prevention or treatment of brain diseases is remarkably effective.

## Description

### TECHNICAL FIELD

This application is based on and claims priority from Korean Patent Application No. 10-2023-0175840, filed on December 6, 2023, with the Korean Intellectual Property Office, the disclosure of which is incorporated herein in its entirety by reference.

The present disclosure relates to an adeno-associated virus variant capable of brain astrocyte-specific gene delivery.

### BACKGROUND ART

Adeno-associated virus (AAV) is a promising gene delivery vector associated with gene therapy. AAV has various serotypes and differs in gene delivery characteristics depending on the amino acid sequence within the hypervariable region of the capsid protein. AAV is non-pathogenic, has low immunogenicity, and can stably express transgenes in episomes. However, the existing AAVs have various tropisms, have limited ability to bypass biological barriers, and may exhibit decreased efficiency due to neutralizing antibodies in the human body.

Brain diseases have become a major global health issue, and various diseases such as Alzheimer's disease, Parkinson's disease, brain tumor, etc. cause functional and structural damage to the brain. The gene therapy technology is increasingly gaining attention for the treatment of these diseases. Gene therapy has the potential to fundamentally resolve the cause of the disease through introduction or inhibition of specific genes.

Hypoxic-ischemic brain injury (HIBI) is a serious brain disease that can occur in people of various ages, including newborns, adults, the elderly, etc. It is a condition in which nerve cells and supporting cells of the brain are damaged due to the lack of oxygen and blood supply to brain tissue. In particular, in newborns, HIBI can occur due to complications during the birth process, and early treatment is essential as this has a high potential to cause permanent neurological disability.

Hypoxic-ischemic brain injury involves complex pathological processes, in which astrocytes play a crucial role in the survival and recovery of nerve cells. Astrocytes play a pivotal role in various aspects, such as regulation of inflammatory responses, protection of nerve cells, promotion of nerve regeneration, etc. at the site of injury. Brain astrocytes play an important role in the maintenance of homeostasis of the nervous system, the regulation of neurotransmitters, and the brain's immune response. However, in hypoxic-ischemic brain injury, the function of the astrocytes is impaired or changes pathologically, which can lead to the worsening of brain damage.

Targeting brain astrocytes to restore or regulate their function is considered an important approach in the treatment of hypoxic-ischemic brain injury. While the existing pharmacological therapies are primarily focused on protecting damaged neurons or suppressing inflammatory responses, they have limitations in effectively regulating specific cell types targeted for treatment, particularly brain astrocytes.

Gene therapy using AAVs is emerging as a promising alternative for the treatment of hypoxic-ischemic brain injury. However, the existing AAV vectors have low selective delivery efficiency to specific cells, leading to problems such as gene delivery to non-target cells (off-targeting) and side effects resulting therefrom. Therefore, there is an urgent need for a technology that can improve the treatment efficiency and reduce side effects of brain diseases, including hypoxic-ischemic brain injury, by developing new adeno-associated virus variants capable of specifically delivering genes to brain astrocytes.

### DISCLOSURE

### Technical Problem

Therefore, the problem that the present disclosure aims to solve is to provide an adeno-associated virus variant capable of specifically delivering genes to brain astrocytes.

### Technical Solution

In order to solve the above problem, the inventors of the present disclosure have made consistent research efforts and discovered a mutant of the adeno-associated virus capsid protein capable of specifically delivering genes to brain astrocytes.

The present disclosure relates to a variant of the adeno-associated virus capsid protein capable of specifically delivering genes to brain astrocytes.

The term 'adeno-associated virus' or AAV used in the present disclosure refers to all adeno-associated viruses used in gene therapy, including their derivatives, viral subtypes, and naturally occurring and recombinant forms. The genomic sequences of various serotypes of AAV, as well as the sequences of natural terminal repeats (TR), Rep proteins, and capsid subunits, are known in the art. For example, the sequences of AAV1 can be referenced in GenBank registry numbers NC_002077 (AAV-1) and AF063497 (AAV-1).

The term 'serotype' used in the present disclosure refers to a subdivision of AAV that can be identified by serological or DNA sequencing methods and distinguished by its antigenic characteristics.

The term capsid used in the present disclosure refers to a protein that constitutes the outer shell of the virus, which is encoded by the cap gene present in the virus genome. The wild-type AAV genome or cap gene codes for three types of capsid proteins (VP1, VP2, and VP3). The wild-type AAV1 capsid VP1 protein contains the amino acid sequence represented by SEQ ID NO: 1.

The present disclosure provides a mutant of the adeno-associated virus serotype 1 (AAV1) capsid protein, wherein the mutant is a mutant of the AAV1 capsid protein in which one or more amino acids located at positions 35, 449, and 451 in the amino acid sequence of SEQ ID NO: 1 of the wild-type AAV1 capsid protein are substituted. In one aspect of the present disclosure, the mutant is a mutant of the AAV1 capsid protein including one or more of the substitution of asparagine (N) at position 35 with aspartic acid (D), the substitution of threonine (T) at position 449 with serine (S), and the substitution of asparagine (N) at position 451 with lysine (K), in the amino acid sequence of SEQ ID NO: 1 of the wild-type AAV1 capsid protein. In one aspect of the present disclosure, the mutant may include all of the N35D, T449S, and N451K substitutions. In the present disclosure, the mutant of the AAV1 capsid protein is named AAV1#11-6, which includes or consists of the amino acid sequence of AAV1#11-6 capsid VP1 represented by SEQ ID NO: 4.

Additionally, the present disclosure provides a mutant of the adeno-associated virus serotype 1 (AAV1) capsid protein, wherein the mutant is a mutant of the AAV1 capsid protein in which one or more amino acids located at positions 237 and 582 in the amino acid sequence of SEQ ID NO: 1 of the wild-type AAV1 capsid protein are substituted. In one aspect of the present disclosure, the mutant is a mutant of the AAV1 capsid protein including one or more of the substitution of aspartic acid (D) at position 237 with asparagine (N) and the substitution of valine (V) at position 582 with aspartic acid (D), in the amino acid sequence of SEQ ID NO: 1 of the wild-type AAV1 capsid protein. In one aspect of the present disclosure, the mutant may include both D237N and V582D substitutions. In the present disclosure, the mutant of the AAV1 capsid protein is named AAV1#11-10, which includes or consists of the amino acid sequence of AAV1#11-10 capsid VP1 represented by SEQ ID NO: 7.

In addition, the present disclosure provides a mutant of the adeno-associated virus serotype 1 (AAV1) capsid protein, wherein the mutant includes one or more substitutions selected from a group consisting of: asparagine (N) at position 35 being replaced with aspartic acid (D) (N35D), threonine (T) at position 449 being replaced with serine (S) (T449S), asparagine (N) at position 451 being replaced with lysine (K) (N451K), aspartic acid (D) at position 237 being replaced with asparagine (N) (D237N), and valine (V) at position 582 being replaced with aspartic acid (D) (V582D), in the amino acid sequence of SEQ ID NO: 1 of the wild-type AAV1 capsid protein. A person skilled in the art to which the present disclosure pertains will readily understand that, considering the superior tropism of the AAV1#11-6 and AAV1#11-10 capsid variants for astrocytes, another mutant of the AAV1 capsid protein including one or more of their amino acid substitution variations will also exhibit equivalent tropism.

In one aspect of the present disclosure, the present disclosure provides a capsid protein mutant of another serotype (e.g., AAV2 to AAV9) having the same variation at a position corresponding to the amino acid variant of the AAV1 capsid protein mutant. The capsid protein mutant of the another serotype may also have superior tropism for brain astrocytes since it includes the variation corresponding to that of the AAV1 capsid protein mutant.

Additionally, the present disclosure provides a nucleic acid encoding the mutant of the AAV1 capsid protein. The nucleic acid of the present disclosure codes for the mutant of the AAV1 capsid protein. The nucleic acid of the present disclosure may exist in the form of DNA, but in some cases, it may be in the form of RNA or a chimera of DNA and RNA. Additionally, the nucleic acid of the present disclosure includes a complementary nucleic acid (e.g., cDNA). The nucleic acid of the present disclosure may be single-stranded or double-stranded. Specifically, it may be double-stranded.

The present disclosure provides a nucleic acid encoding a mutant of the AAV capsid protein consisting of any one of the amino acid sequences of SEQ ID NOS: 4 to 9. Additionally, the present disclosure provides a nucleic acid having a base sequence represented by any one of SEQ ID NOS: 13 to 18.

The nucleic acid of the present disclosure may be operably linked with an appropriate control sequence. The control sequence includes a promoter sequence, a polyadenylation signal, a transcription termination sequence, an upstream regulatory domain, an internal ribosome entry site, an enhancer, etc. The promoter sequence includes an inducible promoter sequence and/or a constitutive promoter sequence. The control sequence may be native to the AAV from which the capsid protein is derived or may be foreign, and may be a natural sequence or a synthetic sequence. A recombinant DNA capable of expressing a mutant of the AAV capsid protein containing the nucleic acid of the present disclosure is also included in the present disclosure.

The recombinant DNA described above is useful for delivering the nucleic acid of the present disclosure to cells in vitro, ex vivo, and in vivo, and for conferring the ability to express a mutant of the AAV capsid protein in the cells. Additionally, the cells to which the nucleic acid of the present disclosure has been delivered are also useful for producing recombinant AAV particles. The recombinant DNA can be used to deliver or introduce the nucleic acid of the present disclosure, particularly to eukaryotic cells, specifically animal cells, and more specifically mammalian cells.

In the present disclosure, a recombinant DNA can be produced by introducing the nucleic acid of the present disclosure into a DNA used as a vector. For example, a plasmid, a phage, a transposon, a cosmid, an episomal DNA, a viral genome, etc., can be used.

For example, a packaging plasmid can be constructed by introducing a nucleic acid (cap gene) encoding a mutant of the AAV capsid protein of the present disclosure into a plasmid. The packaging plasmid may additionally contain any nucleic acid sequence, such as a nucleic acid encoding the replicase (Rep) protein (rep gene), etc.

The method of introducing base substitutions into the nucleic acid can be carried out by known methods and is not particularly limited, but can be achieved by using commercially available reagents, e.g., the mutagenesis basal kit (Takara Bio Inc.), and performing PCR according to the instructions attached to the kit.

The present disclosure provides a recombinant AAV1 vector containing a nucleic acid encoding the AAV capsid protein variant. The recombinant AAV vector is useful for gene introduction into target cells, specifically brain astrocytes. The gene introduced by the recombinant AAV vector of the present disclosure is strongly expressed in the target cells, specifically brain astrocytes.

The term 'AAV vector' as used in the present disclosure refers to any vector containing or derived from the components of adeno-associated virus and suitable for infecting mammalian cells, including human cells, of many tissue types such as brain, heart, lung, skeletal muscle, liver, kidney, spleen, or pancreas, whether in vitro or in vivo. The term 'AAV vector' may be used to refer to an AAV-type virus particle (or virion) containing at least a nucleic acid molecule encoding a protein of interest.

The terms 'AAV virus', 'AAV virus particle', or 'rAAV vector particle' as used in the present disclosure refer to a virus particle consisting of at least one AAV capsid protein (from all capsid proteins of wild-type AAV) and a polynucleotide rAAV vector encapsidated into the capsid. If the particle contains a heterogeneous polynucleotide (i.e., a polynucleotide other than the wild-type AAV genome, e.g., a transgene delivered to a mammalian cell), it is typically referred to as 'rAAV vector particle' or simply an 'rAAV vector'. Thus, the production of rAAV particles inevitably involves the production of rAAV, since such a vector is contained within the rAAV particle.

In one aspect of the present disclosure, a helper virus for AAV refers to a virus that allows AAV (e.g., wild-type AAV) to be replicate and be packaged in mammalian cells. Various helper viruses for AAV, including adenovirus, herpesvirus, and poxvirus such as vaccinia, are known in the art. Although adenovirus type 5 of subgroup C is the most commonly used, adenoviruses include a number of different subgroups. Numerous adenoviruses of human, non-human mammalian, and avian origin are known and are available from depositary institutions such as ATCC.

The function of a helper virus refers to the function encoded in the helper virus genome that allows for AAV replication and packaging (along with other requirements for replication and packaging described herein). As described herein, the helper virus function may be provided in various ways, such as by providing a helper virus or, for example, by providing a polynucleotide sequence encoding an essential function to a producer cell in a trans manner. For example, a plasmid or other expression vector containing a nucleotide sequence encoding one or more adenovirus protein is transfected into a producer cell along with an rAAV vector.

In the present disclosure, the term 'tropism' refers to the specificity of an AAV capsid protein present in an AAV virus particle for infecting or transducing a specific type of cell or tissue. The tropism of an AAV capsid to a specific type of cell or tissue can be determined by measuring the ability of an AAV vector particle, including the AAV capsid protein, to infect or transduce the specific type of cell or tissue using standard analytical methods well known in the art, such as those disclosed in the examples of the present disclosure.

In one aspect of the present disclosure, a mutant of the AAV capsid protein has at least 5%, 10%, 20%, 30%, 40%, 50% or more increased brain astrocyte tropism as compared to that of the wild-type AAV capsid protein.

Additionally, the present disclosure provides a pharmaceutical composition containing the recombinant AAV vector. The composition may further contain a pharmaceutically acceptable carrier. The pharmaceutically acceptable carrier includes any substance that, when combined with the active ingredient of the composition, enables the ingredient to retain biological activity without causing adverse physiological responses, such as unintended immune responses.

In one aspect of the present disclosure, the pharmaceutical composition may be used for the prevention or treatment of brain-related diseases. The brain-related disease may be, but is not limited to, hypoxic-ischemic brain injury, Alzheimer's disease, Parkinson's disease, brain tumor, multiple sclerosis, cerebral palsy, glioblastoma, metastatic brain cancer, encephalitis, autoimmune neurological disease, Alexander disease, etc. Specifically, the brain-related disease may be hypoxic-ischemic brain injury.

In the present disclosure, the 'treatment' means any type of intervention or process performed on a subject or administration of an active agent to a subject for the purpose of reversing, alleviating, improving, inhibiting, delaying, or preventing the progression, development, severity, or recurrence of a disease-related syndrome, complication, symptom, or biochemical sign. The treatment may be performed on a subject with the disease or on a subject without the disease (e.g., for prophylactic purposes).

Additionally, the present disclosure provides a method for preventing or treating a brain-related disease, specifically hypoxic-ischemic brain injury, which includes a step of administering a therapeutically effective amount of the pharmaceutical composition described above to a subject.

In the present disclosure, the term "administration" means physically introducing a therapeutic agent or a composition containing a therapeutic agent into a subject using any of various methods and delivery systems known to those skilled in the art. In the present disclosure, specific routes of administration include intracerebral, intravenous, intraperitoneal, intramuscular, subcutaneous, spinal, airway, vitreous, or other parenteral administration routes, for example, by injection or infusion. The parenteral administration generally means a method of administration other than intestinal and local administration by injection, and is not limited thereto, but includes injection and infusion via intravenous, intraperitoneal, intramuscular, intra-arterial, intrathecal, intralymphatic, intracerebral, intralesional, intracapsular, intraorbital, intracardiac, intradermal, transhepatic, subcutaneous, subepidermal, vitreous, intra-articular, subcapsular, subarachnoid, intraspinal, epidural, airway, or sternal routes. Specifically, in the present disclosure, the administration may be intracerebral, intralesional administration.

In the present disclosure, the term 'therapeutically effective amount' refers to the amount of a drug alone or in combination with another therapeutic agent that is effective in 'treating' a subject's disease or disorder or reducing the risk, potential, likelihood, or occurrence of the disease or disorder (e.g., brain-related disease). The therapeutically effective amount includes the amount of a drug or a therapeutic agent that provides some improvement or benefit to the subject who has or is at risk of having the disease or disorder (e.g., hypoxic-ischemic brain injury). Accordingly, the therapeutically effective amount is an amount that reduces the risk, potential, likelihood, or occurrence of the disease or disorder, or provides some alleviation or relief, and/or reduces at least one indicator and/or reduces at least one clinical symptom of the disease or disorder (e.g., brain-related disease).

In one aspect of the present disclosure, the pharmaceutical composition may be delivered in volumes ranging from about 0.1 mL to about 10 mL, depending on the size of the area to be treated, the viral titer used, and the route and method of administration. The effective concentration of AAV in the pharmaceutical composition may range from about 10⁷ to 10¹⁴ vector genomes per milliliter (vg/mL). To reduce risks such as toxicity, it is preferable to use the lowest effective concentration of virus. The dosage or volume of administration may be selected by an attending physician in consideration of the subject to be treated, specifically body condition, the age of the subject, specific disorder, and, in the case of a progressive disorder, the degree of development of the disorder.

In the present disclosure, the 'subject' includes any human or non-human animal. The non-human animal includes all vertebrates, e.g., mammals and non-human primates, sheep, dogs, cattle, and non-mammalians such as, chickens, amphibians, reptiles, etc.

In addition, the present disclosure encompasses a drug delivery system including the recombinant AAV vector. The composition may further contain a known pharmaceutically acceptable carrier for use in the application described above.

### Advantageous Effects

The present disclosure relates to a recombinant AAV capsid variant capable of specifically targeting brain astrocytes and delivering genes with high efficiency.

When a recombinant viral vector containing a nucleic acid encoding a mutant of the AAV capsid protein of the present disclosure is administered, therapeutic genes can be specifically delivered to and expressed in brain astrocytes, resulting in significantly superior effects in preventing or treating brain disease.

### DESCRIPTION OF DRAWINGS

FIG. 1 shows the 3D modeling of AAV variants. The sites of variation on the surface are indicated in red color.
FIG. 2 shows the distribution of the GFP gene expressed by AAV variants delivered to a hypoxic-ischemic brain injury animal model in Experimental Example 2.
FIG. 3 shows the result of staining cell nuclei with DAPI (blue) using GFAP (red) as an astrocyte-specific marker, and then measuring cell counts and co-expression ratios in Experimental Example 2.
FIG. 4 shows the result of immunohistochemical staining analysis after injection of a NeuroD1-loaded AAV variant in Experimental Example 3.
FIG. 5 shows the result of analyzing GFAP/GFP or NeuN/GFP expression over time after injection of a NeuroD1-loaded AAV variant in Experimental Example 4.
FIG. 6 shows the change in body weight and mortality after injection of a NeuroD1-loaded AAV variant in Experimental Example 5.
FIG. 7 shows the DNA copy number and blood test result depending on tissue type after injection of a NeuroD1-loaded AAV variant in Experimental Example 6.

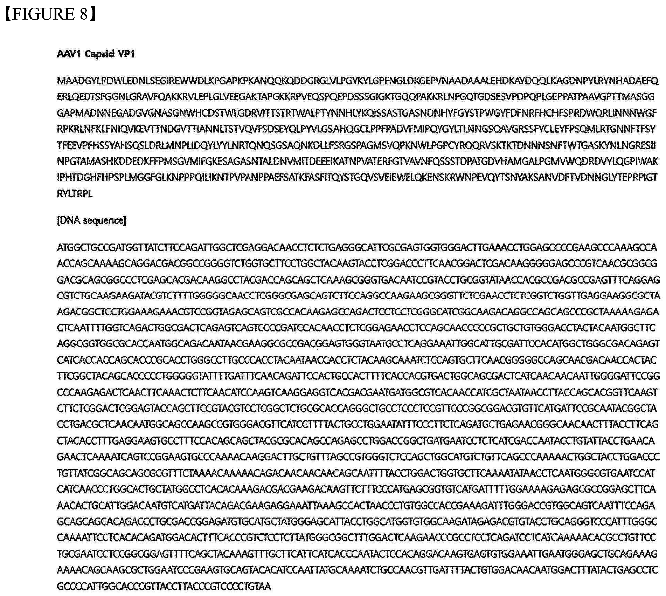

FIG. 8 shows the amino acid sequence and DNA sequence of AAV1 capsid VP1.

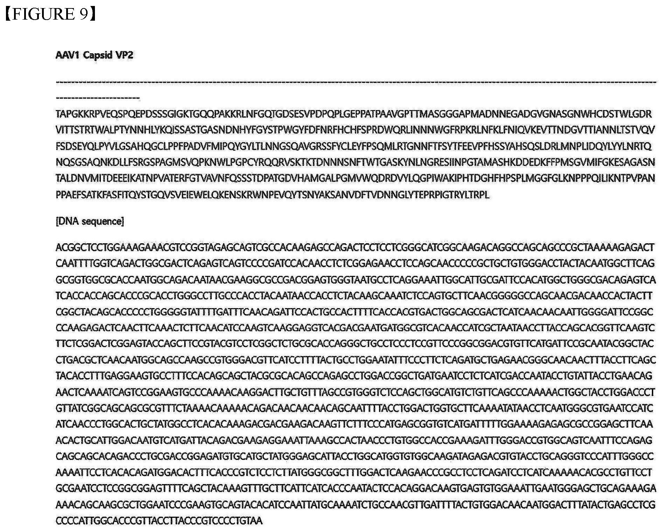

FIG. 9 shows the amino acid sequence and DNA sequence of AAV1 capsid VP2.

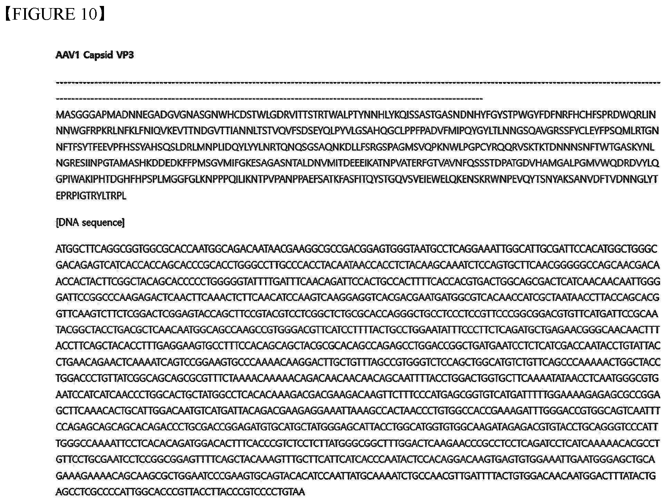

FIG. 10 shows the amino acid sequence and DNA sequence of AAV1 capsid VP3.

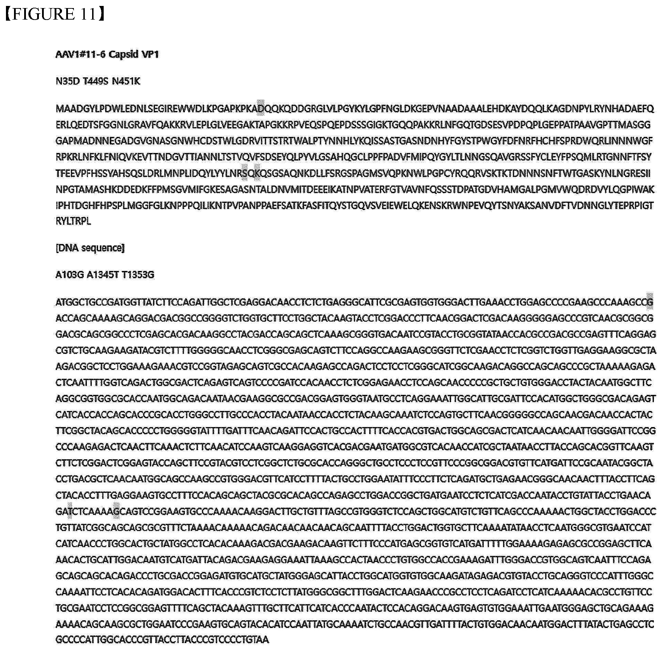

FIG. 11 shows the amino acid sequence and DNA sequence of AAV1#11-6 capsid VP1.

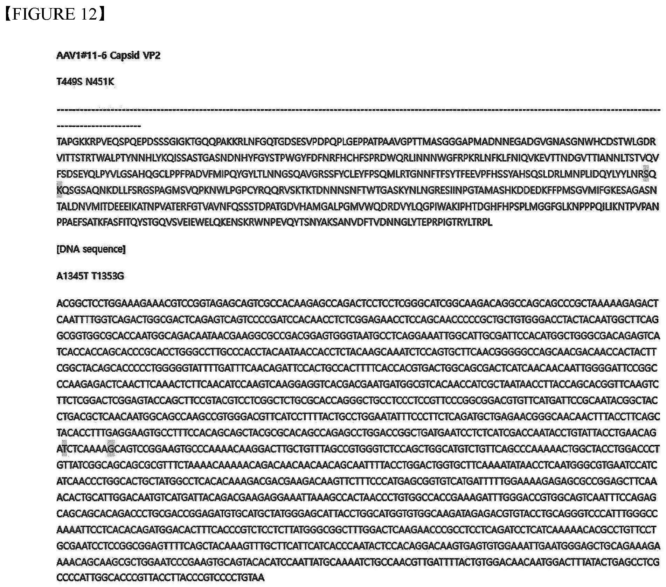

FIG. 12 shows the amino acid sequence and DNA sequence of AAV1#11-6 capsid VP2.

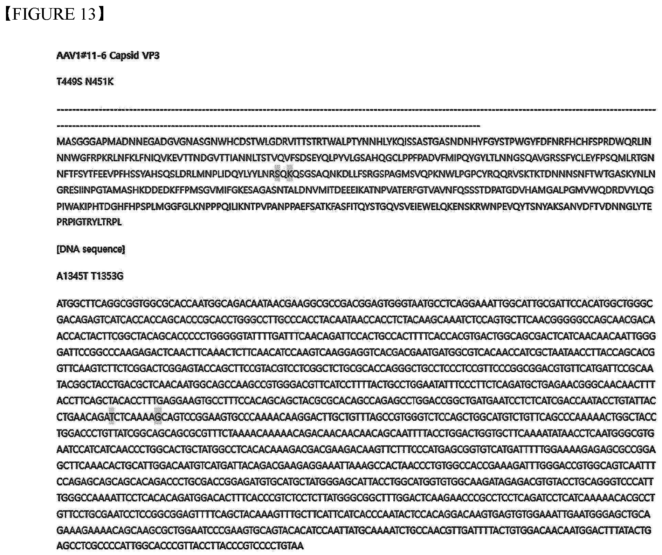

FIG. 13 shows the amino acid sequence and DNA sequence of AAV1#11-6 capsid VP3.

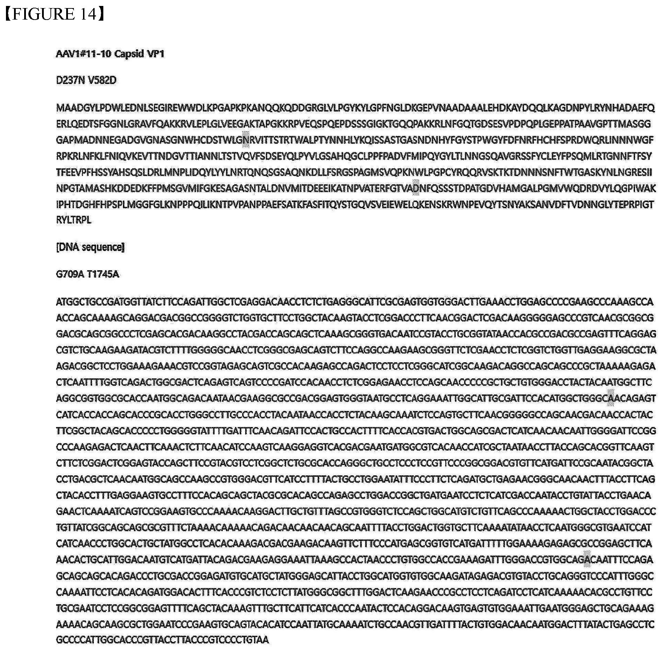

FIG. 14 shows the amino acid sequence and DNA sequence of AAV1#11-10 capsid VP1.

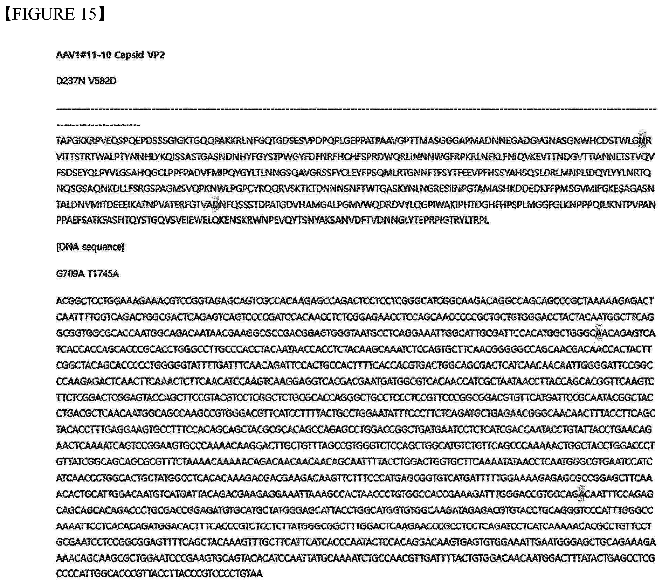

FIG. 15 shows the amino acid sequence and DNA sequence of AAV1#11-10 capsid VP2.

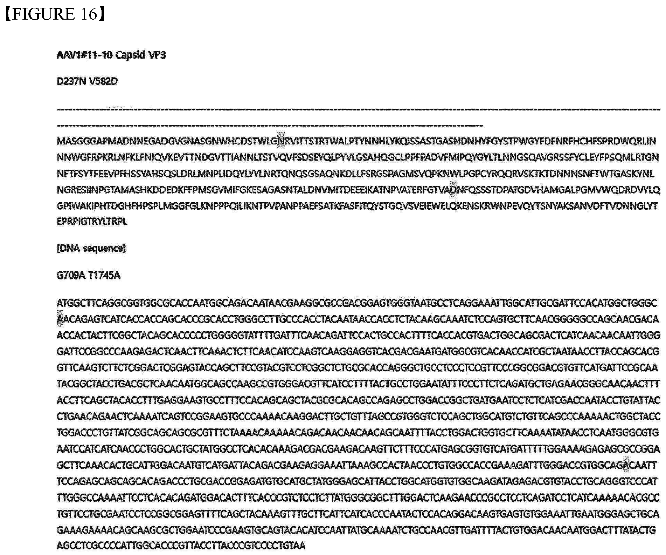

FIG. 16 shows the amino acid sequence and DNA sequence of AAV1#11-10 capsid VP3.

### BEST MODE

Hereinafter, the present disclosure will be described in detail using examples, etc. to aid in the understanding of the present disclosure. However, the examples according to the present disclosure may be modified in various different forms, and the scope of the present disclosure should not be interpreted as being limited to the following examples. The examples of the present disclosure are provided to more fully explain the present disclosure to a person with average knowledge in the field to which the present disclosure belongs.

### <Screening of AAV capsid protein variants>

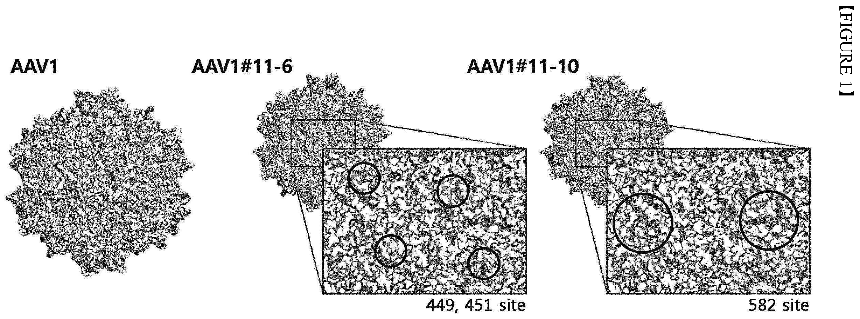

AAV capsid protein variants with tropism for brain astrocytes were screened through *in vivo* directed evolution. Specifically, a library pool was prepared by inducing random point mutations in the cap genes of wild-type AAV variants (AAV1, AAV2, AAV4, AAV6, AAV8, AAV9) using error-prone PCR, and then injected into a hypoxic-ischemic brain injury animal model. After two weeks, each lesion site was isolated, and only astrocytes were separated from the lesions. Viral genes were recovered from the extracted astrocytes, and gene clones were produced. Recombinant AAV variants carrying the GFP fluorescent gene were produced using these gene clones. Subsequently, the recombinant AAV variants were injected into a hypoxic-ischemic brain injury animal model, and two variants capable of inducing high efficiency of gene expression in astrocytes at the hypoxic-ischemic brain injury lesion site were selected (AAV1#11-6, AAV1#11-10). FIG. 1 shows the 3D modeling result of the two variants.

The sequence identification numbers of the amino acid sequences of the capsid variants are shown in Table 1 below.

**[Table 1]**

| Amino acid sequence of capsid | SEQ ID NO |
|---|---|
| AAV1 capsid VP1 | SEQ ID NO: 1 |
| AAV1 capsid VP2 | SEQ ID NO: 2 |
| AAV1 capsid VP3 | SEQ ID NO: 3 |
| AAV1#11-6 capsid VP1 | SEQ ID NO: 4 |
| AAV1#11-6 capsid VP2 | SEQ ID NO: 5 |
| AAV1#11-6 capsid VP3 | SEQ ID NO: 6 |
| AAV1#11-10 capsid VP1 | SEQ ID NO: 7 |
| AAV1#11-10 capsid VP2 | SEQ ID NO: 8 |
| AAV1#11-10 capsid VP3 | SEQ ID NO: 9 |

In addition, the sequence identification numbers of the DNA sequences of capsid variants are shown in Table 2 below.

**[Table 2]**

| DNA sequence of capsid | SEQ ID NO |
|---|---|
| AAV1 capsid VP1 | SEQ ID NO: 10 |
| AAV1 capsid VP2 | SEQ ID NO: 11 |
| AAV1 capsid VP3 | SEQ ID NO: 12 |
| AAV1#11-6 capsid VP1 | SEQ ID NO: 13 |
| AAV1#11-6 capsid VP2 | SEQ ID NO: 14 |
| AAV1#11-6 capsid VP3 | SEQ ID NO: 15 |
| AAV1#11-10 capsid VP1 | SEQ ID NO: 16 |
| AAV1#11-10 capsid VP2 | SEQ ID NO: 17 |
| AAV1#11-10 capsid VP3 | SEQ ID NO: 18 |

### Experimental Example 1: Hypoxic-ischemic brain injury animal model

One-week-old ICR mice were used as an animal model in the laboratory of the Yonsei Biomedical Research Institute, Yonsei University College of Medicine, which is accredited by the AAALAC International (Association for Assessment and Accreditation of Laboratory Animal Care International). The carotid artery leading to the right brain of the animal model was ligated to sever blood flow, and brain injury was induced by exposing the animal to a hypoxic environment (8% O₂ and 90% N₂). Viruses were injected into the lesion site during the acute phase (3 days after the spinal cord injury). Subsequently, blood was removed by perfusion while adjusting the sacrifice date depending on the gene expression time of the injected viruses. In the subsequent experiment, brain tissue was either isolated immediately or obtained after fixing the tissue with 4% PFA.

### Experimental Example 2: Immunohistochemical (IHC) comparison of AAV1#11-6, AAV1#11-10 and wild-type AAV1

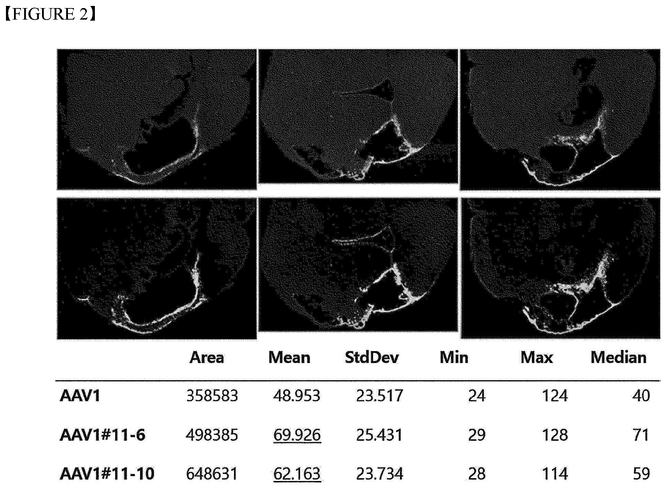

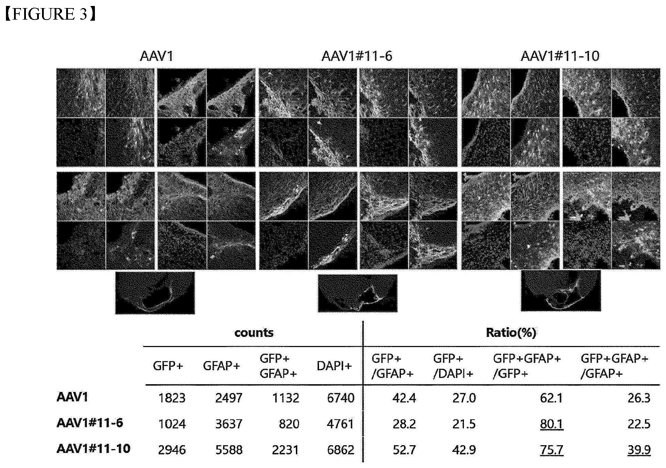

AAV1#11-6, AAV1#11-10, and wild-type AAV1 (1x10⁸ vg/µL; 6 µL) were injected into the lesion site during the acute phase after the hypoxic-ischemic brain injury. The injected viruses carried the GFP gene, and the expression of the GFP gene could be confirmed through immunohistochemical staining. Tissues were isolated on the 7th day after the AAV injection, and the distribution of AAV expression around the lesion site was analyzed through immunohistochemical staining using GFP and GFAP (FIG. 2). Additionally, cell counts and co-expression ratios were measured for each marker using the astrocyte-specific marker GFAP (red) and the cell nucleus staining marker DAPI (blue). The result is shown in FIG. 3. Based on the experimental result, it was confirmed that AAV1-based AAV1#11-6 and AAV1#11-10 showed higher gene delivery efficiency to the lesion site as compared to the wild-type AAV1.

### Experimental Example 3: Analysis of astrocyte infection efficiency of AAV variants

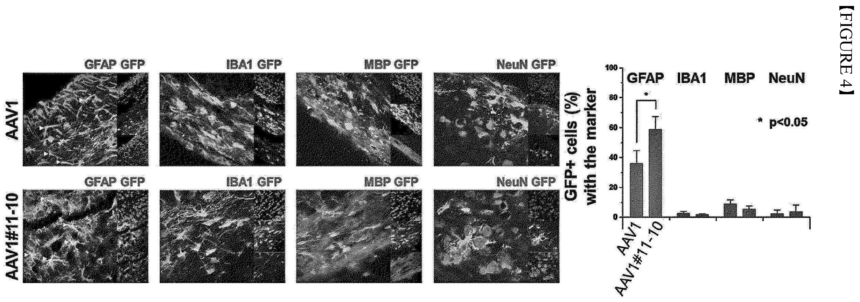

The distribution of gene expression was examined for AAV1#11-10, which showed a high GFP expression rate from among the AAV variants selected from the hypoxic-ischemic brain injury animal model, and the parent vector, AAV1. On the 3rd day after the induction of the brain injury model, the two variants loaded with CMV-NeuroD1-T2A-GFP and the parent vector, AAV1, were injected into astrocytes. One week after the injection, the expression distribution of the variants was analyzed through immunohistochemical staining. The expression of the astrocyte marker (GFAP), microglial marker (IBA1), oligodendrocyte marker (MBP), and neuronal marker (NeuN) was confirmed in GFP-positive cells. The result of the immunohistochemical staining analysis after the injection of the NeuroD1-loaded AAV variants is shown in FIG. 4.

The AAV1#11-10 variant showed high ratios as 57.1% for GFAP, 1.6% for IBA1, 5.5% for MBP, and 3.8% for NeuN. Meanwhile, AAV1 showed ratios of 35.8% for GFAP, 2.6% for IBA1, 8.9% for MBP, and 2.4% for NeuN. Among the GFP-positive cells, the cells that accounted for the highest proportion were astrocytes, and the increased infection of astrocytes by the AAV1#11-10 variant with a significant statistical difference (p < 0.05) from to the parent vector, AAV1, was confirmed.

### Experimental Example 4: Analysis of induction of direct transdifferentiation by AAV variants

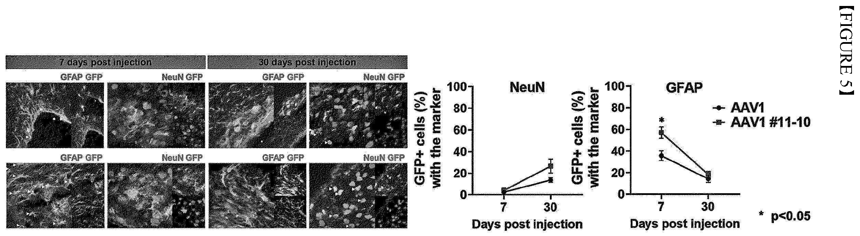

To confirm the efficiency of dedifferentiation of astrocytes into neurons by NeuroD1 after gene expression, CMV-NeuroD1-T2A-GFP-loaded AAV1#11-10 and AAV1 were transplanted during the acute phase after the hypoxic-ischemic brain injury. Immunohistochemical staining was performed 7 and 30 days after the transplantation to analyze the expression of astrocytes (GFAP+) or neurons (NeuN+) in the AAV-infected cells (GFP+) over time after the transplantation, thereby analyzing the direct transdifferentiation efficiency. FIG. 5 shows the result of analyzing GFAP/GFP or NeuN/GFP expression at different times after the injection of the NeuroD1-loaded AAV variants.

It was confirmed that astrocytes were transdifferentiated into neurons because the proportion of astrocytes decreased and the proportion of neurons increased among the AAV-infected cells. For the AAV1#11-10 variant, GFAP decreased from 57.1% (day 7) to 18.6% (day 30), and NeuN increased from 3.8% (day 7) to 26.7% (day 30). Meanwhile, for AAV1, GFAP decreased from 35.8% (day 7) to 14.3% (day 30), and NeuN increased from 2.4% (day 7) to 13.6% (day 30).

### Experimental Example 5: Analysis of safety of AAV variants in vivo

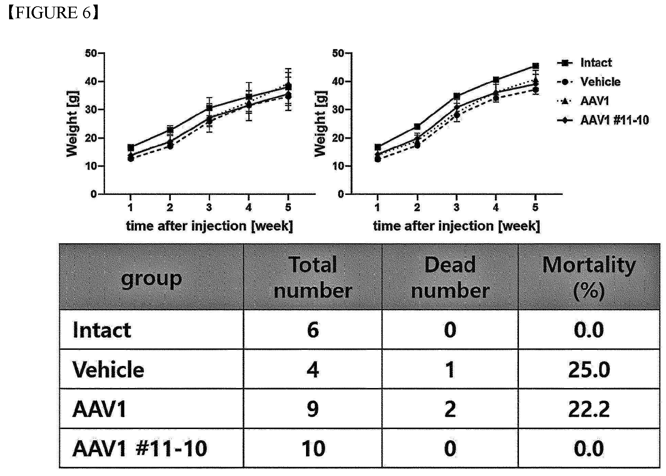

After transplanting CMV-NeuroD1-T2A-GFP-loaded AAV1 and AAV1#11-10 variants, or saline (vehicle group) during the acute phase after the hypoxic-ischemic brain injury, in-vivo safety was observed by measuring mortality and body weight of the animals. FIG. 6 shows the change in body weight and mortality of the animal models after the injection of the NeuroD1-loaded AAV variants. The mortality rate after the viral transplantation was highest in the vehicle group at 25%, while all individuals survived for the AAV1#11-10 variant. No statistically significant difference in body weight was found between the groups after the transplantation.

### Experimental Example 6: Off-target and toxicity tests for AAV variants

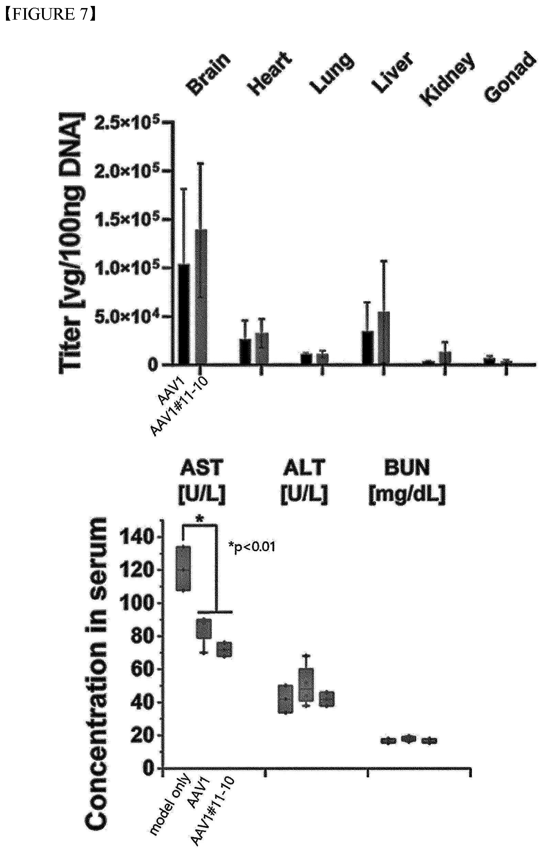

As in Experimental Example 6 described above, tissues (brain, heart, lung, liver, kidney, gonads) of each group were isolated at 4 weeks after the transplantation of each virus. After extracting DNA from each isolated tissue, copy number analysis of the transferred genes and blood test were performed. FIG. 7 shows the DNA copy number and blood test result for each tissue after the injection of the NeuroD 1-loaded AAV variant. The highest copy number was observed in the brain, followed by the liver and heart, and there was no statistically significant difference as compared to the parent vector. In the blood test, it was confirmed that the AST level, which indicates hepatotoxicity, was statistically significantly increased in the vehicle group as compared to the groups injected with AAV.

## Claims

1. A mutant of the adeno-associated virus serotype 1 (AAV1) capsid protein, wherein the mutant is a mutant of the AAV1 capsid protein in which one or more amino acids located at positions 237 and 582 in the amino acid sequence of the wild-type AAV1 capsid protein (SEQ ID NO: 1) are substituted.

2. The mutant of the AAV1 capsid protein according to claim 1, wherein the mutant comprises one or more of the substitution of aspartic acid (D) at position 237 with asparagine (N) and the substitution of valine (V) at position 582 with aspartic acid (D), in the amino acid sequence of the wild-type AAV1 capsid protein (SEQ ID NO: 1).

3. The mutant of the AAV1 capsid protein according to claim 2, wherein the mutant comprises both the substitution of aspartic acid (D) at position 237 with asparagine (N) and the substitution of valine (V) at position 582 with aspartic acid (D), in the amino acid sequence of the wild-type AAV1 capsid protein (SEQ ID NO: 1).

4. A nucleic acid encoding the mutant of the AAV1 capsid protein according to claim 1.

5. The nucleic acid according to claim 4, wherein the nucleic acid has a base sequence represented by SEQ ID NO: 16.

6. A recombinant AAV1 vector comprising the nucleic acid according to claim 4 or 5.

7. The recombinant AAV1 vector according to claim 6, wherein the recombinant AAV1 vector has superior tropism for brain astrocytes when compared with an AAV1 wild-type viral vector.

8. A pharmaceutical composition for preventing or treating a brain disease, comprising the recombinant AAV1 vector according to claim 6.

9. The pharmaceutical composition for preventing or treating a brain disease according to claim 8, wherein the brain disease is hypoxic-ischemic brain injury.
